# EUROPEAN PATENT SPECIFICATION

(11) **EP 0 908 177 B1**
(45) Date of publication and mention of the grant of the patent: **21.04.2004**
(21) Application number: 98500202.1
(22) Date of filing: 11.09.1998
(51) Int. Cl.: A61K 9/50, A61K 31/55

(54) **Procedure to form pharmaceutical compositions in spherical pills or granules of controlled and sustained action that contain as active agent Bromazepam and pharmaceutical compositions obtained by said procedure**
Verfahren zur Herstellung von Bromazepam enthaltende pharmazeutische Zusammensetzungen in Form von kugelformigen Pillen oder Granulaten zur kontrollierter und verzögerter Freigabe und hergestellte Zusammensetzungen
Procédé pour former des compositions pharmaceutiques de bromazépam sous forme de granules ou pilules sphériques à action controlée et soutenue et compositions pharmaceutiques obtenues par ce procédé

(30) Priority: 12.09.1997 AR 1004185
(43) Date of publication of application: 14.04.1999
(73) Proprietor: Bindecor Sociedad Anonima, Montevideo (UY)
(72) Inventor: Gold, Oscar, 1069 Buenos Aires (AR)
(74) Representative: Garcia-Cabrerizo y del Santo, Pedro Maria

(56) References cited:
- EP-A- 0 335 560
- DE-A- 3 430 389
- US-A- 3 864 486

## Description

### Scope of the invention

The present invention is related to a composition of substantially spherical granules or pills of controlled and sustained action, capable to maintain clinically acceptable therapeutic levels of 7-bromo-1,3-dihydro-5-(2-pyridinyl)-2H-1,4-benzodiazepin-2(1H)-one(Bromazepam) with a single daily dose, enough to form stable plasmatic concentrations and effective levels during twenty-four hours.

The mentioned compound (7-bromo-1,3-dihydro-5-(2-pyridinyl) -2H-1,4-benzodiazepin-2(1H)-one) is at the moment used in medications in their conventional, quick or immediate forms, for the treatment of emotional disorders whose symptomatology is expressed by means of states of anxiety, anguish, obsession, compulsions, phobias and hypochondrias, as it has positive effects on the psychic stresses, anxiety, nervousness and it also has sedative and myorelaxant properties. A procedure to prepare said composition is also proposed.

### Background of the Invention

Bromazepam or 7-bromo-1,3-dihydro-5-(2-pyridinyl)-2H-1,4-benzodiazepin-2(1H)-one) is known as from 1962, Belgian Patent N° 619.101 (1962), U.S. Patent N° 3.100.770 (1963), N° 3.182.065 and N° 3.182.067 (1964).

In these documents Bromazepam and analogue compounds are described, as well as the preparation of the same and their application for the treatment of altered emotional states, by means of pharmaceutical formulations of conventional or quick action doses of 1,5 mg, 3mg, and 6 mg.

In the world market there exist a great variety of pharmaceutical compositions of controlled and sustained action in granulated form or pills. Within this diversity there are certain suitable products for the treatment of emotional disorders that are advantageous, because they allow to have medicines of controlled and sustained action with which the number of daily doses is reduced, thus offering a greater safety and effectiveness and additionally therapeutic levels during longer periods, just as it happens for example with the dipotasic chlorazepate, diazepam, etc. administered in doses of controlled and sustained release.

In this aspect, it has been seen now that it is possible to prepare pharmaceutical compositions of controlled and sustained effects advantageously, which are particularly apt for the treatment of altered psychic states, related or associated with disorders or emotional flaws, by means of a treatment that implies the administration of formulations that have different release levels in vivo of Bromazepam - specifically a quick release and another of slow but sustained release - with which serious sustained and constant but long levels are obtained that in practices it means a better and more constant bioavailavility of Bromazepam with a smaller number of daily doses.

### Description of the invention

According with the present invention, compositions of controlled and sustained action formed by pills or substantially spherical granules that contain (7-bromo-1,3-dihydro-5-(2-pyridinyl)-2H-1,4-benzodiazepin-2 (1H) -one) (active agent) that are released in a sustained way maintaining stable plasmatic concentrations, are proposed.

Said compositions contain (7-bromo-1,3-dihydro-5-(2-pyridinyl)-2H-1,4-benzodiazepin-2(1H)-one) including spherical granules (indistinctly mentioned hereinafter) as nodules or neuter (made up of sugar and starch), formed by following steps:
1. The incorporation of the micronized active agent on the nodules or neuter through polymers in solution, forming the substantially spherical granules that are generally denominated quick action granules or immediate action granules.
2. Polymers are added to a fraction of the product obtained in the previous stage, which polymers adhere and that come from a solution configuring some spheres of controlled and sustained action of the active principle.
3. Combination, association or blend of the pills emergent from stage 1 with the granules of controlled and sustained action of stage 2. The nodules have a size around 0.2 mm to 1.8 mm preferably around 0.4 mm to 1.5 mm.

The solutions used for the obtaining of the pills or substantially spherical (or rounded) granules of quick action or release are, for example, acetone, isopropylic alcohol, ethylic alcohol, chloroform, methylene chloride, water or blend of the same and although the present invention does not depend on specific bonding polymers, polyvinylpyrrolidones, polyethylenglycols, methylcellulose, saccharose, jellys, starch and mixtures thereof are preferred.

In an embodiment of this invention, once the granules of immediate action or release are dry, a fraction of the same is processed to form a second polymeric coating on the first, to form granules or pills of slow release or action or controlled and sustained action.

The solvents used in the preparation of one and the other coatings are preferably acetone, isopropylic alcohol, ethylic alcohol or other alcohol, chloroform, methylene chloride, water or a blend thereof.

The polymers can be such as to form the mentioned coating with whole polymers such as the polymers and copolymers of the methacrylic acid (different types of eudragit L, Eudragit RL or Eudragit RS or combinations among them), cellulose acetophthalate, different types of hydroxypropylmethylcelluloses phthalate, hydroxypropylmethylcelluloses, ethylcelluloses, shellac, etc. these polymers can be combined in different proportions among them.

It is possible to incorporate diethylphthalate, polyethylenglycol, triethylcitrate, triethylcitrate, triacetine, triglycerics of fatty acids, or other plasticizers and before the drying it the incorporation of lubricant is convenient, just as talc that additionally reduces the agglutination or binding of the particles.

Preferably, in the second stage (formation of the mentioned coating) the following proportions are recommended:
- Active agents - Bromazepam - the proportion with relation to the total amount of exicipients will be from 0.5 to 15% and that of the exicipients and binding polymers with regard to the rest of materials from 0.5 to 7%.
- According to the invention, it is preferred that the final concentration of the active agent in the pills or granules to be from 0.5 to 14% and that the diameter of those in the order of 0.5 mm to 1.9 mm, preferably between 0.7 mm and 1.8 mm.
- Preferred aspects of the invention, are given by the following ratios: when ethylcelluloses are used, it is advisable a final concentration of up to 50%.
- Solvents used in stages 1 and 2 can be formulated within a wide range of possibilities, extended between the applications of anhydrous solvents and the exclusive application of water.
- The proportion of pills or granules of quick or immediate action of the stage 1 with regard to the final mixture is included between zero and 50% ponderal.
- The pills or granules that contain (7-bromo-1,3-dihydro-5-(2-pyridinyl)-2H-1,4-benzodiazepin-2(1H)-one) according to the present invention are administrable in different doses, in capsules of hard jelly at concentrations preferably from 1 mg to 18 mg.

These pills or granules are dissolved in a controlled and sustained way and this action can be verified "in vitro" with the team of Breakup type XXIII USP pages 1791 Apparatus II of the palette type at 502 r.p.m. and each glass with 800 ml of artificial gastric juice taking samples in different hourly reactions.

The breakup profile of these granules of (7-bromo-1,3-dihydro-5-(2-pyridinyl) -2H-1,4-benzodiazepin-2(1H)-one) is the following:

| | |
|---|---|
| 1st. hour | 20 % - 50 % |
| 4th. hour | 50 % - 85 % |
| 8th. hour | > 80 % |

In accordance with this invention dosages of (7-bromo-1,3-dihydro-5-(2-pyridinyl) -2H-1,4-benzodiazepin-2(1H)-one) of sustained and controlled action with stable plasmatic levels are obtained.

In the following illustrative examples, it is demonstrated how the present invention can be carried out.

### Example 1

| | |
|---|---|
| Neuter (sugar/starch nodules) | 190.0 g. |
| Polyvinylpyrrolidone | 2.8 g. |
| Polyethylenglycol | 3.0 g. |
| Eudragit L | 2.0 g. |
| Eudragit RL | 2.0 g. |
| Ethylcellulose | 3.0 g. |
| Triethylcitrate | 1.0 g. |
| Bromazepam | 6.0 g. |
| Talc | 14.0 g. |

1. The active agent is micronized to a size less than 40 microns (10-6 meters).
2. The support material is incorporated under the form of spherical nodules or particles (denominated neuters) into a shallow bowl of stainless steel that rotates at a speed between 7 and 35 r.p.m. and the active agent is incorporated simultaneously, by means of spraying on the same a solution that also contains; polyvinylpyrrolidone, polyethylenglycol in isopropylic alcohol at 10% (solids/liquids p/w).
   Once the process is finished the product is allowed to dry off.
3. From the material processed and formed in the stage 2, a 30% ponderal is separated, and the remaining mass (70% ponderal) remains in the shallow bowl and at a speed between 7 and 35 r.p.m. it is treated by spraying with a solution that contains (Eudragit L, Eudragit RL, ethylcellulose triethylcitrate) to 10% p/w dissolved in 90% of a solvent means of 60% isopropylic alcohol, 30% acetone and 10% water.
4. The product obtained from stage 3 is dried up and before being treated with (anti-agglutinant) talc it is blended with the granules remainders from stage 2.

### Example 2

| | |
|---|---|
| Neuter (sugar/starch nodules) | 205.0 g |
| Polyvinylpyrrolidone | 5.0 g. |
| Methylcellulose | 1.0 g |
| Cellulose Acetophthalate | 1.0 g |
| Eudragit RS | 1.0 g |
| Ethylcellulose | 1.0 g |
| Diethylphthalate | 0.8 g |
| Bromazepam | 6.0 g |
| Talc | 10.0 g |

1. The active agent is micronized to a size less than 40 microns.
2. The spheroidal nodules(neuter) are incorporated to a shallow bowl of stainless steel that rotates at a speed between 7 and 35 r.p.m.
   The active agent is added simultaneously by spraying on said granules or neuter of a solution of said active agent in a solvent means including: polyvinylpyrrolidone, methylcellulose and cellulose acetophthalate dissolved to 8% p/w of solid/liquid, in a mixture of 90% isopropylic alcohol and 10% water.
   Once the process is finished, the resulting product is allowed to dry.
3. From the granules formed and recovered from stage 2, a mass equivalent to 33% is separated, and the remaining 67% is retained in the shallow bowl and at a speed between 7 and 35 r.p.m., it is treated by means of spraying with a solution that contains (Eudragit RS, ethylcellulose, dibuthylphthalate) total 8% p/w dissolved in 92% of a solvent mixture of 55% isopropylic alcohol, 40% acetone and 5% water.
4. The product formed in stage 3 is dried up, and previously treated with talc, and it is blended with the granules formed in the stage 2.

### Example 3

| | |
|---|---|
| Neuter (sugar/starch) | 186.0 g |
| Polyvinylpyrrolidone | 6.0 g |
| Eudragit L | 6.0 g |
| Ethylcellulose | 4.0 g |
| Bromazepam | 6.0 g |
| Talc | 12.0 g |

1. The active agent is micronized to a size less than 40 microns.
2. The nodules are incorporated to a shallow bowl of stainless steel that rotates at a speed between 7 and 35 r.p.m.
   The active agent is added simultaneously, by spraying said agent over said granules or neuter in a solvent solution comprising polyvinylpirrolidone, methylcellulose and cellulose acetophthalate dissolved with a proportion of 8 % solid/liquid, in a blend of 90 % isopropylic alcohol and 10 % water.
   Once the spraying process is finished, the resulting product is allowed to dry.
3. From the granules formed and recovered in the stage 2, a fraction that corresponds to 30% ponderal is separated and the remaining 70% retained in the shallow bowl and at a speed between 7 and 35 r.p.m., is treated by means of spraying with a solution that contains (Eudragit L, ethylcellulose) 10% total p/w dissolved in 90% of a solvent media that includes 60% isopropylic alcohol and 40% acetone.
4. The product formed in the stage 3, previously treated with talc (antiagglutinant) is left to dry and is blended with the remianing granules of stage 2.

### Example 4

| | |
|---|---|
| Neuter (sugar/starch) | 202.0 g |
| Polyvinylpyrrolidone | 3.0 g |
| Polyethylenglycol | 3.0 g |
| Hydroxypropylmethylcellulose Phthalate | 3.0 g |
| Ethylcellulose | 5.0 g |
| Triacetine | 0.5 g |
| Bromazepam | 6.0 g |
| Talc | 9.0 g |

1. The active agent is micronized to a size less than 40 microns
2. The nodules are incorporated to a shallow bowl of stainless steel that rotates at a speed between 7 and 35 r.p.m. and the active agent is incorporated and simultaneously a solution of polyvinylpyrrolidone, polyethylenglycol 9% p/w in a solvent media is added by spraying, said solvent comprising 95% of isopropylic alcohol and 5% water.
   Once the process is finished the resulting product is allowed to dry.
3. From the coated granules formed in stage 2, a mass equivalent to 34% of the total is separated and the remaining mass (66% of the total retained in the shallow bowl at a speed between 7 and 35 r.p.m.) is treated by spraying a solution of hydroxypropylmethylcellulose phthalate ethylcellulose and triacetine to 7% in total p/w dissolved in a solvent mixture /93% comprising 80% of isopropanol, 10% of acetone and the rest to a hundred is water.
4. The product formed in the stage 3, is left to dry, being previously treated as in the previous examples with talc and then blended with the remaining mass of granules from stage 2.

### Example 5

| | |
|---|---|
| Neuter (sugar/starch) | 198.0 g |
| Polyvinylpyrrolidone | 5.0 g |
| Methylcellulose | 1.0 g |
| Hydroxypropylmethylcellulose | 1.0 g |
| Eudragit L | 6.0 g |
| Ethylcellulose | 3.0 g |
| Polyethylenglycol | 0.4 g |
| Bromazepam | 11.0 g |
| Talc | 11.0 g |

1. The active agent is micronized to a size less than 40 microns.
2. The spherical nodules (or neuter) are incorporated to a shallow bowl of stainless steel that rotates at a speed between 7 and 35 r.p.m., being simultaneously treated by spraying of a solution of the active agent 8% ponderal with a solution of PVP and methylcellulose - 8% ponderal in a solvent media that sprays a solution on the spheres denominated neutres;said solution including isopropanol, 90% and water 10%.
3. From the dry product formed in the previous stage, an equivalent fraction to 35% ponderal is separated (65%) retained in the shallow bowl and at a speed between 7 and 35% r.p.m. it is treated by spraying a solution of 8% p/w hydroxypropylmethylcelluloses, Eudragit L, ethylcellulose, polyethylenglycol 92% p/w of a solvent mixture that includes 90% isopropanol, 5% water.
4. The product formed and recovered in the stage 3 is dried, being previously treated as in the previous examples with talc and being blended with the remainder mass separated in stage 2.

With the samples taken in these examples the tests of dissolution of the granules according to USP XXIII blades method was carried out with 800 ml of artificial gastric juice in each glass and at 50 r.p.m. with samples taken at certain intervals (column 1). The results are illustrated in the following chart:

| Percentage of Release | | | | | |
|---|---|---|---|---|---|
| Hs. | Example 1 | Example 2 | Example 3 | Example 4 | Example 5 |
| 1 | 31 % | 35 % | 30 % | 37 % | 35 % |
| 4 | 72 % | 74 % | 68 % | 72 % | 71 % |
| 8 | 91 % | 94 % | 90% | 96% | 95 % |

Although in the present invention a reference is made to the nodules or nuclei formed by sugar/starch (that is to say nodules formed by a coherent mass of particles of sugar agglutinated with starch) it must be bom in mind that other compounds may be applied to formulate said nuclei subject to the condition of pharmaceutically acceptable: other mono or polysacharids, natural gums and in the case of insuline-dependent patients, lipids or proteins.

On the other hand, the pills or granules to form the compositions of the present invention can be formulated with granules of quick release and granules of sustained release, formed sequentially or formulated with granules of one and the other type prepared separatedly, stored and then gathered "in situ", given the case of being used independently and according to the clinical decision of the case.

### Pharmacokinetic study

In accordance with the previously described and the present examples, capsules that contained 9 mg of 7-bromo-1,3-dihydro-5-(2-pyridinyl) -2H-1,4-benzodiazepin-2 (1H-ona) of sustained and controlled action were presented and comparative studies with capsules that contained 3mg of 7-bromo-1,3dihydro-5-(2-pyridinyl)-2H-1,4-benzodiazepin-2(1H)-one) and with capsules that contained 1,5 mg of 7-bromo-1,3-dihydro-5-(2-pyridinyl)-2H-1,4-benzodiazepin-2-(1H)-one) were carried out.

The studies were carried out with 10 healthy volunteers and was administered oral via. The method was crossed with a washing time of 2 weeks between each formulation. The plastic concentrations were analyzed and the statistical study of the parameters was evaluated.

The products are defined as follows:
LC 3mg: Capsules of conventional or immediate action that contain 3 mg of 7-bromo-1,3-dihydro-5-(2-pyridinyl)-2H-1,4-benzodiazepin-2(1H)-one).
LP 9mg: Capsules with granules or pills of controlled and sustained action that contain 7-bromo-1,3-dihydro-5-(2-pyrinyl)-2H-1,4-benzodiazepin-2(1H)-one)
LC 1,5 mg: Capsules of conventional or immediate action that contain 1,5 mg of 7-bromo-1,3-dihydro-5-(2-pyridinyl)-2H-1,4-benzodiazepin-2(1H)-one).

The administration is defined in the following way:

| Formulation | Dose | Number |
|---|---|---|
| LC 3mg | 3 mg. | 1 |
| LP 9mg. | 9 mg. | 1 |
| LC 1,5 mg | 1,5 mg | 6 (1 every 90 min.) |

The characteristics associated with the formulation LP 9 mg in relation with Lc 3 mg and LC 1,5 mg 6 intakes 1 every 90 min. are the following:

The product LP 9 mg has stable plasmatic levels in form of plateau between the 2nd. and 12th. hour with a TMAX at the 8th hour and with active values at 24 hours and if we compare with product LC 1,5 mg 6 intakes 1 every 90 min. it has a Tmax a Cmax and AUC with non significant differences among them and with a similar plateau between the 6th and 12th hours and similar values at 24 hours.

With regard to the formulation LC 3mg the AUC is approximately three times but this product has a Tmax and a Cmax at first hour and starting from this some descending and not very significant levels at 24 hours.

Therefore the product LP 9mg. is defined as of controlled and sustained action showing a displacement of the Tmax and Cmax with regard to the LC 3mg, but a triple AUC; at the same time being similar to the AUC, TMAX and Cmax of the LC 1,5 mg 6 intakes at a rate of 1 each 90 ' and concentrations stable in the following intervals.

Table 1 shows the parameters of LC 3 mg, LP 9 mg and LC 1,5 mg 6 intakes 1 every 90 min., and the average concentrations in time on 10 volunteers.

**Table 1**

| Formulation/Parameters | Cmax (ng/ml) | Tmax (hours) | Auc 0-24 (ng.h/ml) |
|---|---|---|---|
| LC 3 mg | 38.1 | 1 | 320.8 |
| LP 9 mg | 48.0 | 8 | 961.3 |
| LC 1,5 mg | | | |
| 6 takings 1 per 90' | 50.0 | 8 | 929.5 |

Having thus specially described and determined the nature of the present invention, and how it can be carried out, the following is claimed as of exclusive right and property:

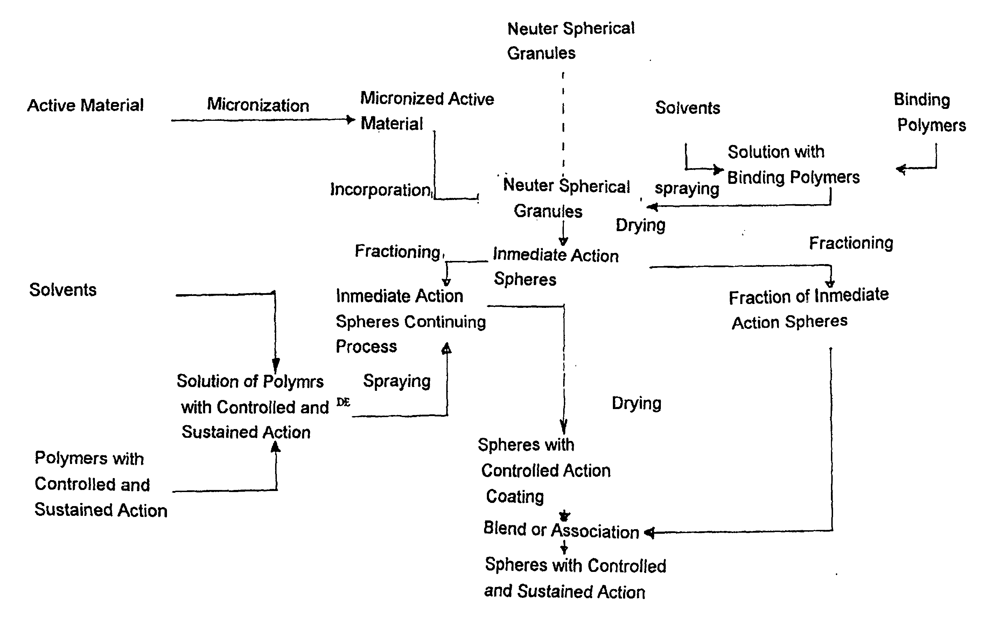

## Claims

1. Process to form pharmaceutical compositions in substantially spherical pills or granules of controlled and sustained action that contain as active agent 7-bromo-1,3-dihydro-5-(2-pyridinyl)-2H-1,4-benzodiazepin-2(1H)-one (Bromazepam), **characterized in that** it comprises the following stages:
a) a coating of nodules or nuclei formed by particles of sugar consolidated with starch, in an operation of blending of said nodules with micronized Bromazepam and a bonding polymer in solution, to form a biodegradable coating on said nodules, which retains the micronized material, forming the group of coated nodules, a first formulation, of quick release of Bromazepam;
b) forming on at least one separated fraction of the released nodules coming from stage a), a second coating, in an operation of blending of said fraction with a film-forming enteric polymer in solution forming said fraction provided of this enteric coating in solution, a second formulation, of slow release;
c) combining enough quantities of formulations of Bromazepam of quick release and of slow or sustained release formed in the stages a) and b), to form compositions that contain predetermined doses and of programmed release of Bromazepam.

2. Process in accordance with claim 1, **characterized in that** the active agent is micronized to a size less than 40 µm (microns).

3. Process in accordance with claim 1, **characterized in that** the nodules or nuclei have a diameter from 0,2 to 1,8 mm preferably between 0,4 to 1,5 mm.

4. Process in accordance with claim 1, **characterized in that** the bonding polymers that form the first coating are selected among polyvinylpyrrolidones (PVP), polyethylenglycols, methylcellulose, saccharose, jelly, starch and combinations thereof.

5. Process in accordance with claim 1, **characterized in that** the polymers forming the second coating, with polymers of enteric dissolution such as polymers and copolymers of esters of methacrylic acid cellulose acetophthalate, hydroxypropylmethylcellulose phthalate, hydroxypropylmethylcellulose or shellac and combinations thereof.

6. Process in accordance with claim 1, **characterized in that** the percentage of nodules of quick action in combination with the nodules of controlled and sustained action is from 0% to 50%.

7. Process in accordance with claim 1, **characterized in that** the solvents used in stages a) and b) are chosen among acetone, isopropylic alcohol, ethylic alcohol, chloroform, methylene chloride, water or combinations thereof.

8. Process in accordance with claim 1, **characterized in that** the solution contains plasticizers such as diethylphthalate, dibutylphthalate triacetine triethylcitrate triglycerids of fatty acids, alone or a combination thereof

9. Process in accordance with claim 1 **characterized in that** the proportion of active agent with regard to the rest of the excipients is from 0,5% to 15%.

10. Process in accordance with claims 1 and 2 **characterized in that** the proportion of bonding polymers with regard to the remaining components is from 0,5 to 7%.

11. Process in accordance with claims 1 and 2 **characterized in that** the ethylcellulose proportion in the final concentration is from 0% to 50%.

12. Process in accordance with claim 1 and **characterized in that** the final concentration of active agent in the nodules is from 0,5% to 14%.

13. Process in accordance with claims 1 and 2 **characterized in that** the final size of the granules is from 0,6 mm to 2 mm preferably between 0,8 mm to 1,7mm.

14. Process in accordance with claims 1 and 2 **characterized in that** the preparations for the administration of the nodules of controlled and sustained action in capsules of hard jelly, in different concentrations of the active agent 7-bromo-1,3-dihydro-5(2-pyridinyl)-2H-1,4-benzodiazepin-2(1H)-one, are obtained preferably between 1 mg to 18 mg.

15. Process in accordance with claim 1 **characterized in that** the dissolution of the nodules "in vitro" according to the dissolution apparatusUSP XXIII blade type with 800 ml of artificial gastric juice at 50 r.p.m. and at 37° corresponds with this profile:
1 st. hour 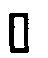 20% - 50%
4th. hour 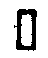 50% - 85%
8th. hour― 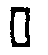 > 80%.

16. Process in accordance with claims 1 and 2, **characterized in that** the nodules of extended and sustained action obtained, maintain plasmatic concentrations Cmax and Tmax as compared with the conventional administration in a single intake and at the same time similar concentrations to a same doses of the administration in form of conventional action in repeated numbers; therefore the spheres showing a controlled and sustained active action of 7-bromo-1,3-dihydro-5(2-pyridinyl)-2H-1,4-benzodiazepin-2(1H)-one.

17. Pharmaceutical compositions in substantially spherical pills or granules, with controlled and sustained release of 7-bromo-1,3-dihydro-5(2-pyridiny))-2H-1,4-benzodiazepin-2(1H)-one as active agent, resulting from the process of claim 1, **characterized in that** they include predetermined proportions of substantially spherical pills or granules of:
i) a formulation of quick release including nodules or nuclei formed by particles of sugar consolidated with starch coated with a biodegradable coating formed by a film-forming bonding polymer and fixed on these nodules that includes the micronized active agent;
ii) a second formulation of slow release including said nodules or coated nuclei, provided with a second coating, formed by a film-forming enteric polymer and fixed on the same coating, the relationship between the first one and second formulation in both compositions being selected for predetermined doses and of programmed release of Bromazepam.

18. Pharmaceutical compositions in accordance with claim 17, **characterized in that** the active agent is micronized to a size of less than 40 µm (microns).

19. Pharmaceutical compositions in accordance with claim 17, **characterized in that** the nodules or nuclei have a diameter from 0,2 to 1,8 mm preferably between 0,4 to 1,5 mm.

20. Pharmaceutical compositions in accordance with claim 17, **characterized in that** the bonding polymers that form the first coating are selected from polyvinylpyrrolidones (PVP), polyethylenglycols, methylcellulose, saccharose, jelly, starch and combinations thereof.

21. Pharmaceutical compositions in accordance with claim 17, **characterized in that** the polymers form the second coating, with polymers of enteric dissolution such as polymers and copolymers of esters of methacrylic acid cellulose acetophthalate, hydroxypropylmethylcellulose phthalate, hydroxypropylmethylcellulose or shellac and combinations thereof.

22. Pharmaceutical compositions in accordance with claim 17, **characterized in that** the percentage of nodules of quick action in the combination with the nodules of controlled and sustained action is from 0% to 50%.

23. Pharmaceutical compositions in accordance with claim 17, **characterized in that** the solvents used in the stages a) and b) are chosen from acetone, isopropylic alcohol, ethylic alcohol, chloroform, methylene chloride, water or combinations thereof.

24. Pharmaceutical compositions in accordance with claim 17, **characterized in that** the solution contains plasticizers such as diethylphthalate, dibutylphthalate triacetine triethylcitrate triglycerids of fatty acids, alone or a combination thereof.

25. Pharmaceutical compositions in accordance with claim 17, **characterized in that** the proportion of active agent with regard to the rest of the excipients is from 0,5% to 15%.

26. Pharmaceutical compositions in accordance with claim 17, **characterized in that** the proportion of bonding polymers with respect to the remaining components is from 0,5 to 7%.

27. Pharmaceutical compositions in accordance with claim 17, **characterized in that** the proportion of ethylcellulose in the final concentration is from 0% to 50%.

28. Pharmaceutical compositions in accordance with claim 17, **characterized in that** the final concentration of the active agent in the nodules is from 0,5% to 14%.

29. Pharmaceutical compositions in accordance with claim 17, **characterized in that** the final size of the granules is from 0,6 mm to 2 mm, preferably between 0,8 mm and 1,7 mm.

30. Pharmaceutical compositions in accordance with claim 17, **characterized in that** they include preparations for the administration of the nodules of controlled and sustained action in capsules of hard jelly, in the different concentrations of the active agent 7-bromo-1,3-dihydro-5(2-pyridinyl)-2H-1,4-benzodiazepin-2(1H)-one, preferably among 1 mg to 18mg.

31. Pharmaceutical compositions in accordance with claim 17, **characterized in that** the dissolution of the nodules "in vitro" according to the breakup apparatus USP XXIII of blade type with 800 ml of artificial gastric juice at 50 r.p.m. and at 37° corresponding with this profile:
1 st. hour 20% - 50%
4th. hour 50% - 85%
8th. hour > 80%.

32. Pharmaceutical compositions in accordance with claim 17, **characterized in that** the nodules of extended and sustained action obtained, keep plasmatic concentrations Cmax and Tmax in comparison with the conventional administration in a single intake and at the same time similar concentrations to same dosage of the administration in form of conventional action in repeated intakes; the spheres therefore showing a controlled and sustained action of active matter 7-bromo-1,3-dihydro-5(2-pyridinyl)-2H-1,4-benzodiazepin-2(1H)-one.

## Patentansprüche

1. Verfahren zur Herstellung pharmazeutischer Verbindungen in Pillen- oder Granulatform, im Wesentlichen kugelförmig, mit kontrollierter und anhaltender Wirkung, die 7-Brom-1,3-dihydro-5-(2-pyridinil)-2-H-1,4-benzodiazepin-2(1H)-ona (Bromazepam) als aktiven Bestandteil enthalten, **dadurch gekennzeichnet, dass** es die folgenden Etappen umfasst:
a) eine Beschichtung aus Kügelchen oder Kernen aus Zuckerpartikeln, über Stärke befestigt, in einer Mischoperation besagter Kügelchen mit zerkleinertem Bromazepam und einem verbindenden Polymer in Lösung, um eine biologisch abbaubare Beschichtung über besagten Kügelchen zu erhalten, die das zerkleinerte Material zurückhält, unter Bildung einer Gruppe beschichteter Kügelchen, einer ersten Formulierung mit schneller Freisetzung des Bromazepam;
b) Bildung, wenigstens in einem getrennten Teil der freigesetzten Kügelchen aus der Etappe a), einer zweiten Beschichtung in einer Mischoperation besagten Teils mit einem darmlöslichen Polymer, das in Lösung einen Film bildet, wobei besagter Anteil mit dieser darmlöslichen Beschichtung in Lösung eine zweite Formulierung mit langsamer Freisetzung bildet;
c) eine Kombination der hinreichenden Mengen an Formulierungen mit schneller und langsamer oder anhaltender Freisetzung, die in den Etappen a) und b) hergestellt worden sind, um Verbindungen zu bilden, die eine vorbestimmte Dosis und Zusammensetzungen mit programmierter Freisetzung von Bromazepam enthalten.

2. Verfahren nach Anspruch 1, **dadurch gekennzeichnet, dass** der aktive Bestandteil bis auf eine Größe unter 40 µm (Mikrometer) zerkleinert wird.

3. Verfahren nach Anspruch 1, **dadurch gekennzeichnet, dass** die Kügelchen oder Kerne einen Durchmesser zwischen 0,2 und 1,8 mm, vorzugsweise zwischen 0,4 und 1,5 mm aufweisen.

4. Verfahren nach Anspruch 1, **dadurch gekennzeichnet, dass** die verbindenden Polymere, die die erste Beschichtung bilden, ausgewählt werden aus Polyvinylpyrrolidonen (PVP), Polyethylenglykolen, Methylzellulosen, Saccharose, Gelatine, Stärke und Kombinationen derselben.

5. Verfahren nach Anspruch 1, **dadurch gekennzeichnet, dass** die Polymere, die die zweite Beschichtung bilden, darmlösliche Polymere sind, wie die Polymere und Kopolymere der Ester aus Metacrylzellulosacetophtalat, Phtalate der Hydroxypropylmethylzellulose, Hydroxypropylmethylzellulose oder Schellack und Kombinationen derselben.

6. Verfahren nach Anspruch 1, **dadurch gekennzeichnet, dass** der Prozentanteil der Kügelchen mit schneller Wirkung in Verbindung mit den Kügelchen mit kontrollierter und anhaltender Wirkung zwischen 0% und 50% liegt.

7. Verfahren nach Anspruch 1, **dadurch gekennzeichnet, dass** die in den Etappen a) und b) verwendeten Lösungsmittel aus Azeton, Isopropylalkohol, Ehtylalkohol, Chloroform, Chlormethylen, Wasser oder Kombinationen derselben ausgewählt werden.

8. Verfahren nach Anspruch 1, **dadurch gekennzeichnet, dass** die Lösung Weichmacher enthält wie Triglyzeride mit Diethylphtalat, Dibutylphtalat, Triazetin oder Triethylcitrat aus Fettsäuren, einzeln oder in Kombination.

9. Verfahren nach Anspruch 1, **dadurch gekennzeichnet, dass** der Prozentsatz an aktivem Bestandteil bezüglich der restlichen Auflösungsmittel zwischen 0,5% und 15% liegt.

10. Verfahren nach den Ansprüchen 1 und 2, **dadurch gekennzeichnet, dass** der Prozentsatz an verbindenden Polymeren bezüglich der restlichen Komponenten zwischen 0,5% und 7% liegt.

11. Verfahren nach den Ansprüchen 1 und 2, **dadurch gekennzeichnet, dass** der Prozentsatz der Ethylzellulose in der Endkonzentration zwischen 0% und 50% liegt.

12. Verfahren nach Anspruch 1, **dadurch gekennzeichnet, dass** die Endkonzentration an aktivem Bestandteil in den Kügelchen zwischen 0,5% und 14% liegt.

13. Verfahren nach den Ansprüchen 1 und 2, **dadurch gekennzeichnet, dass** die Endgröße der Granulate zwischen 0,6 mm und 2 mm, vorzugsweise zwischen 0,8 mm und 1,7 mm liegt.

14. Verfahren nach den Ansprüchen 1 und 2, **dadurch gekennzeichnet, dass** die Präparate für die Verabreichung der Kügelchen mit kontrollierter und anhaltender Wirkung in Kapseln aus harter Gelatine, mit verschiedenen Konzentrationen des aktiven Wirkstoffs 7-Brom-1,3-dihydro-5-(2-pyridinil)-2-H-1,4-benzodiazepin-2(1H)-ona, vorzugsweise zwischen 1 mg und 18 mg erhalten werden.

15. Verfahren nach Anspruch 1, **dadurch gekennzeichnet, dass** die Lösung der Kügelchen "in vitro" gemäß dem Lösungsapparat USP XXIII mit Messern und 800 ml künstlichem Verdauungssaft bei 50 U/Min. und 37° dem folgenden Profil entspricht:
erste Stunde 20% - 50%
vierte Stunde 50% - 85%
achte Stunde > 80%

16. Verfahren nach den Ansprüchen 1 und 2, **dadurch gekennzeichnet, dass** die erhaltenen Kügelchen mit langer und anhaltender Wirkung die Konzentrationen Cmax und Tmax im Plasma beibehalten, wenn sie mit der konventionellen Verabreichung mit einer einzigen Einnahme verglichen werden, und ähnliche Konzentrationen bei mehrmaliger Verabreichung derselben Dosis in der Form mit konventioneller Wirkung; daher zeigen die Kugeln eine kontrollierte und anhaltende Wirkung von 7-Brom-1,3-dihydro-5-(2-pyridinil)-2-H-1,4-benzodiazepin-2(1H)-ona.

17. Pharmazeutische Verbindungen in Pillen- oder Granulatform, im Wesentlichen kugelförmig, mit kontrollierter und anhaltender Wirkung von 7-Brom-1,3-dihydro-5-(2-pyridinil)-2-H-1,4-benzodiazepin-2(1H)-ona als aktivem Bestandteil, die aus dem Verfahren nach Anspruch 1 stammen, **dadurch gekennzeichnet, dass** sie vorbestimmte Prozentsätze an vorzugsweise kugelförmigen Pillen oder Granulaten enthalten:
i) eine Formulierung mit schneller Freisetzung, die Kügelchen oder Kerne enthält, die aus Zuckerpartikeln hergestellt sind, mit Stärke gebunden, mit einer biologisch abbaubaren Beschichtung aus einem verbindenden Polymer, das filmbildend ist, fixiert über diesen Kügelchen, die den zerkleinerten aktiven Bestandteil enthalten;
ii) eine zweite Formulierung mit langsamer Freisetzung, die besagte beschichtete Kügelchen oder Kerne enthält und die mit einer zweiter Beschichtung versehen sind, die aus einem darmlöslichen Polymer bestehen, das Filme bilden kann und die an der Schicht selbst befestigt sind, wobei das Verhältnis zwischen der ersten und zweiten Formulierung in beiden Verbindungen für eine vorbestimmte Dosis und die programmierte Freisetzung des Bromazepams ausgewählt wird.

18. Pharmazeutische Verbindungen nach Anspruch 17, **dadurch gekennzeichnet, dass** der aktive Bestandteil auf eine Größe von unter 40 µm (Mikrometer) zerkleinert wird.

19. Pharmazeutische Verbindungen nach Anspruch 17, **dadurch gekennzeichnet, dass** die Kügelchen oder Kerne einen Durchmesser zwischen 0,2 und 1,8 mm, vorzugsweise zwischen 0,4 und 1,5 mm aufweisen.

20. Pharmazeutische Verbindungen nach Anspruch 17, **dadurch gekennzeichnet, dass** die verbindenden Polymere, die die erste Beschichtung bilden, ausgewählt werden aus Polyvinylpyrrolidonen (PVP), Polyethylenglykolen, Methylzellulose, Saccharose, Gelatine, Stärke und Verbindungen derselben.

21. Pharmazeutische Verbindungen nach Anspruch 17, **dadurch gekennzeichnet, dass** die Polymere, die die zweite Beschichtung bilden, darmlösliche Polymere darstellen, wie die Polymere und Kopolymere der Ester mit Metacrylzelluloseazetophtalat, Phtalate der Hydroxypropylmethylzellulose, Hydroxypropylmethylzellulose oder Schellack, und die Kombinationen derselben.

22. Pharmazeutische Verbindungen nach Anspruch 17, **dadurch gekennzeichnet, dass** der Prozentsatz an Kügelchen mit schneller Wirkung in Kombination mit den Kügelchen mit kontrollierter und anhaltender Wirkung zwischen 0% und 50% liegt.

23. Pharmazeutische Verbindungen nach Anspruch 17, **dadurch gekennzeichnet, dass** die in den Etappen a) und b) verwendeten Lösungsmittel ausgewählt werden zwischen Aceton, Isopropylalkohol, Ethylalkohol, Chloroform, Chlormethylen, Wasser oder Kombinationen derselben.

24. Pharmazeutische Verbindungen nach Anspruch 17, **dadurch gekennzeichnet, dass** die Lösung Weichmacher enthält wie Triglyzeride mit Dethylphtalat, Dibutylphtalat, Triazetin oder Triethylzitrat aus Fettsäuren, einzeln oder in Kombination.

25. Pharmazeutische Verbindungen nach Anspruch 17, **dadurch gekennzeichnet, dass** der Prozentsatz an aktivem Bestandteil bezüglich der restlichen Auflösungsmitteln zwischen 0,5% und 15% liegt.

26. Pharmazeutische Verbindungen nach Anspruch 17, **dadurch gekennzeichnet, dass** der Prozentsatz an verbindenden Polymeren bezüglich der restlichen Komponenten zwischen 0,5% und 7% liegt.

27. Pharmazeutische Verbindungen nach Anspruch 17, **dadurch gekennzeichnet, dass** der Prozentsatz an Ethylzellulose in der Endkonzentration zwischen 0% und 50% liegt.

28. Pharmazeutische Verbindungen nach Anspruch 17, **dadurch gekennzeichnet, dass** die Endkonzentration an aktivem Bestandteil in den Kügelchen zwischen 0,5% und 14% liegt.

29. Pharmazeutische Verbindungen nach Anspruch 17, **dadurch gekennzeichnet, dass** die Endgröße der Granulate zwischen 0,6 mm und 2 mm liegt, vorzugsweise zwischen 0,8 mm und 1,7 mm.

30. Pharmazeutische Verbindungen nach Anspruch 17, **dadurch gekennzeichnet, dass** sie Präparate enthalten für die Verabreichung der Kügelchen mit kontrollierter und anhaltender Wirkung in harten Gelatinekapseln, mit verschiedenen Konzentrationen an dem aktiven Bestandteil 7-Brom-1,3-dihydro-5-(2-pyridinil)-2-H-1,4-benzodiazepin-2(1H)-ona, vorzugsweise zwischen 1 mg und 18 mg.

31. Pharmazeutische Verbindungen nach Anspruch 17, **dadurch gekennzeichnet, dass** die "in vitro" Lösung der Kügelchen gemäß dem Lösungsapparat USP XXIII mit Messern und 800 ml künstlichem Verdauungssaft bei 50 U/Min. und 37° dem folgenden Profil entspricht:
erste Stunde 20% - 50%
vierte Stunde 50% - 85%
achte Stunde > 80%

32. Pharmazeutische Verbindungen nach Anspruch 17, **dadurch gekennzeichnet, dass** die erhaltenen Kügelchen mit ausgedehnter und anhaltender Wirkung bei wiederholten Versuchen die Konzentrationen Cmax und Tmax im Plasma beibehalten, wenn sie mit der konventionellen Verabreichung mit einer einzigen Einnahme verglichen werden, und ähnliche Konzentrationen bei mehrmaliger Verabreichung derselben Dosis in der Form mit konventioneller Wirkung; daher zeigen die Kugeln eine aktive, kontrollierte und anhaltende Wirkung durch 7-Brom-1,3-dihydro-5-(2-pyridinil)-2-H-1,4-benzodiazepin-2(1H)-ona.

## Revendications

1. Méthode pour la préparation de compositions pharmaceutiques sous forme de pilules ou granulés essentiellement sphériques à action contrôlée et prolongée, contenant du 7-bromo-1,3-dihydro-5-(2-pyridinyl)-2-H-1,4-benzodiazépine-2(1H)-one (Bromazépam) comme agent actif, **caractérisée en ce qu'**elle présente les étapes suivantes:
a) un enrobage des nodules ou noyaux constitués de particules de sucre consolidé avec de l'amidon, obtenu par le mélange desdits nodules avec du Bromazépam micronisé et un polymère liant par dissolution, pour former un enrobage biodégradable recouvrant lesdits nodules et contenant la matière micronisée, le groupe de nodules enrobés formant une première formulation de Bromazépam à libération rapide;
b) formation, sur au minimum une fraction séparée des nodules libérés à l'issu de l'étape a), d'un second enrobage, en procédant au mélange de ladite fraction avec un polymère entérique capable de former des pellicules par dissolution, ladite fraction étant recouverte de cet enrobage entérique par dissolution et formant une deuxième formulation, à libération lente ;
c) association de quantités suffisantes de formulations de Bromazépam à libération rapide et lente ou prolongée obtenues au cours des étapes a) et b), pour préparer des compositions qui contiennent des doses prédéterminées et des compositions de Bromazépam à libération programmée.

2. Méthode selon la revendication 1, **caractérisée en ce que** l'agent actif est micronisé jusqu'à ce qu'il atteigne une taille inférieure à 40 µm (microns).

3. Méthode selon la revendication 1, **caractérisée en ce que** les nodules ou noyaux présentent un diamètre compris entre 0,2 et 1,8 mm, de préférence entre 0,4 et 1,5 mm.

4. Méthode selon la revendication 1, **caractérisée en ce que** les polymères liants formant le premier enrobage sont sélectionnés parmi le polyvinyle pyrrolidone (PVP), le polyéthylène glycol, la méthylcellulose, la saccharose, la gélatine, l'amidon et les associations de ces derniers.

5. Méthode selon la revendication 1, **caractérisée en ce que** les polymères formant le deuxième enrobage sont des polymères à dissolution entérique comme les polymères et copolymères d'esters d'acétophtalate de cellulose méthacrylique acide, le phtalate d' hydroxypropylméthilcellulose, l'hydroxypropylméthilcellulose ou la gomme laque, et les associations de ces derniers.

6. Méthode selon la revendication 1, **caractérisée en ce que** le pourcentage de nodules à action rapide associés aux nodules à action contrôlée et prolongée est compris entre 0% et 50%.

7. Méthode selon la revendication 1, **caractérisée en ce que** les dissolvants utilisés aux étapes a) et b) sont sélectionnés parmi l'acétone, l'alcool isopropylique, l'alcool éthylique, le chloroforme, le chlorure de méthylène, l'eau ou les associations de ces derniers.

8. Méthode selon la revendication 1, **caractérisée en ce que** la dissolution contient des plastifiants comme les triglycérides, le phtalate de diéthyle, le phtalate de dibutyle, la triacétine ou le citrate de triéthyle des acides gras, seuls ou associés entre eux.

9. Méthode selon la revendication 1, **caractérisée en ce que** la proportion d'agent actif par rapport aux autres excipients est comprise entre 0,5% et 15%.

10. Méthode selon les revendications 1 et 2, **caractérisée en ce que** la proportion de polymères liants par rapport aux autres composants est comprise entre 0,5% et 7%.

11. Méthode selon les revendications 1 et 2, **caractérisée en ce que** la proportion d'éthylcellulose présente dans la concentration finale est comprise entre 0% et 50%.

12. Méthode selon la revendication 1, **caractérisée en ce que** la concentration finale d'agent actif présent dans les nodules est comprise entre 0,5% et 14%.

13. Méthode selon les revendications 1 et 2, **caractérisée en ce que** la taille finale des granulés est comprise entre 0,6 mm et 2 mm, de préférence entre 0,8 mm et 1,7 mm.

14. Méthode selon les revendications 1 et 2, **caractérisée en ce que** les préparations pour l'administration des nodules à action contrôlée et prolongée sous forme de capsules en gélatine dure, avec différentes concentrations d'agent actif 7-bromo-1,3-dihydro-5-(2-pyridinyl)-2-H-1,4-benzodiazépine-2(1H)-one, sont de préférence réalisées entre 1 mg et 18 mg.

15. Méthode selon la revendication 1, **caractérisée en ce que** la dissolution des nodules « in vitro » conformément à l'appareil de dissolution à palettes USP XXIII, avec 800 ml de suc gastrique artificiel à 50 r.p.m. et à 37°, présente ce profil:
première heure 20% - 50%
quatrième heure 50% - 85%
huitième heure > 80%

16. Méthode selon les revendications 1 et 2, **caractérisée en ce que** les nodules à action large et prolongée que l'on obtient, maintiennent les mêmes concentrations plasmatiques Cmax et Tmax que pour une action conventionnelle à des doses équivalentes administrées en plusieurs fois, par comparaison à l'administration conventionnelle en une seule prise et au même moment; en conséquence de quoi les sphères révèlent une action active contrôlée et prolongée du 7-bromo-1,3-dihydro-5-(2-pyridinyl)-2-H-1,4-benzodiazépine-2(1H)-one.

17. Compositions pharmaceutiques sous forme de pilules ou granulés essentiellement sphériques à libération contrôlée et prolongée de 7-bromo-1,3-dihydro-5-(2-pirydinyl)-2-H-1,4-benzodiazépine-2(1H)-one comme agent actif, résultant de la méthode de la revendication 1, **caractérisées en ce qu'**elles comprennent des proportions prédéterminées de pilules ou granulés essentiellement sphériques issues d'une:
i) formulation à libération rapide où des nodules ou noyaux sont formés par des particules de sucre, consolidées avec de l'amidon recouvert d'un enrobage biodégradable formé par un polymère liant capable de former des pellicules, et fixées sur ces nodules, incluant l'agent actif micronisé ;
ii) une deuxième formulation à libération lente, où lesdits nodules ou noyaux enrobés présentent un deuxième enrobage formé par un polymère entérique capable de former des pellicules, et se fixent sur ce même enrobage, et où la relation entre la première et la deuxième formulation dans les deux compositions est choisie pour des doses prédéterminées et des doses de Bromazépam à libération programmée.

18. Compositions pharmaceutiques selon la revendication 17, **caractérisées en ce que** l'agent actif est micronisé jusqu'à ce qu'il atteigne une taille inférieure à 40 µm (micromètres).

19. Compositions pharmaceutiques selon la revendication 17, **caractérisées en ce que** les nodules ou noyaux présentent un diamètre compris entre 0,2 et 1,8 mm, de préférence entre 0,4 et 1,5 mm.

20. Compositions pharmaceutiques selon la revendication 17, **caractérisées en ce que** les polymères liants formant le premier enrobage sont sélectionnés parmi le polyvinyle pyrrolidone (PVP), le polyéthylène glycol, la méthylcellulose, la saccharose, la gélatine, l'amidon et les associations de ces derniers.

21. Compositions pharmaceutiques selon la revendication 17, **caractérisées en ce que** les polymères formant le deuxième enrobage sont des polymères à dissolution entérique comme les polymères et les copolymères d'esters d'acétophtalate de cellulose méthacrylique acide, le phtalate d' hydroxypropylméthilcellulose, l'hydroxypropylméthilcellulose ou la gomme laque, et les associations de ces derniers.

22. Compositions pharmaceutiques selon la revendication 17, **caractérisées en ce que** le pourcentage de nodules à action rapide associés aux nodules à action contrôlée et prolongée est compris entre 0% et 50%.

23. Compositions pharmaceutiques selon la revendication 17, **caractérisées en ce que** les dissolvants utilisés au cours des étapes a) et b) sont sélectionnés parmi l'acétone, l'alcool isopropylique, l'alcool éthylique, le chloroforme, le chlorure de méthylène, l'eau ou les associations de ces derniers.

24. Compositions pharmaceutiques selon la revendication 17, **caractérisées en ce que** la dissolution contient des plastifiants comme les triglycérides, le phtalate de diéthyle, le phtalate de dibutyle, la triacétine ou le citrate de triéthyle des acides gras, seuls ou associés entre eux.

25. Compositions pharmaceutiques selon la revendication 17, **caractérisées en ce que** la proportion d'agent actif par rapport aux autres excipients est comprise entre 0,5% et 15%.

26. Compositions pharmaceutiques selon la revendication 17, **caractérisées en ce que** la proportion de polymères liants par rapport aux autres composants est comprise entre 0,5% et 7%.

27. Compositions pharmaceutiques selon la revendication 17, **caractérisées en ce que** la proportion d'éthylcellulose dans la concentration finale est comprise entre 0% et 50%.

28. Compositions pharmaceutiques selon la revendication 17, **caractérisées en ce que** la concentration finale d'agent actif dans les nodules est comprise entre 0,5% et 14%.

29. Compositions pharmaceutiques selon la revendication 17, **caractérisées en ce que** la taille finale des granulés est comprise entre 0,6 mm et 2 mm, de préférence entre 0,8 mm et 1,7 mm.

30. Compositions pharmaceutiques selon la revendication 17, **caractérisées en ce qu'**elles présentent des préparations pour l'administration des nodules à action contrôlée et prolongée sous forme de capsules de gélatine dure, à différentes concentrations de l'agent actif 7-bromo-1,3-dihydro-5-(2-pirydinyl)-2-H-1,4-benzodiazépine-2(1H)-one, de préférence entre 1 mg et 18 mg.

31. Compositions pharmaceutiques selon la revendication 17, **caractérisées en ce que** la dissolution des nodules "in vitro" selon l'appareil de dissolution à palettes USP XXIII, avec 800 ml de suc gastrique artificiel à 50 r.p.m. et à 37°, présente ce profil:
première heure 20% - 50%
quatrième heure 50% - 85%
huitième heure > 80%

32. Compositions pharmaceutiques selon la revendication 17, **caractérisées en ce que** les nodules à action large et prolongée que l'on obtient maintiennent les mêmes concentrations plasmatiques Cmax et Tmax que pour l'action conventionnelle à des doses équivalentes administrées en plusieurs fois, par comparaison à l'administration conventionnelle en une seule prise et au même moment; en conséquence de quoi les sphères révèlent une action active contrôlée et prolongée du 7-bromo-1,3-dihydro-5-(2-pyridyl)-2-H-1,4-benzodiazépine-2(1H)-one.
